# EUROPEAN PATENT APPLICATION

(11) **EP 1 661 581 A1**
(43) Date of publication of application: **31.05.2006**
(21) Application number: 04748228.6
(22) Date of filing: 28.07.2004
(51) Int. Cl.: A61K 45/00, A61K 31/7105, A61K 31/711, A61K 38/45, A61K 39/395, A61K 48/00, A61P 9/04, G01N 33/15, G01N 33/50

(54) **REMEDY FOR CARDIAC FAILURE COMPRISING ASK1 INHIBITOR AS THE ACTIVE INGREDIENT AND METHOD OF SCREENING THE SAME**

(30) Priority: 28.07.2003 JP 2003281281
(71) Applicant: Osaka Industrial Promotion Organization, Osaka-shi, Osaka 540-0029 (JP)
(72) Inventor: OTSU, Kinya,c/o Osaka Univer.Graduate School of Me, Osaka 565-0871 (JP); NISHIDA,Kazuhiko,c/o Osaka Uni.Graduate School of, Osaka 565-0871 (JP); YAMAGUCHI, Osamu, c/o Osaka.University Graduate, Osaka 565-0871 (JP); HIGUCHI, Yoshiharu, c/o Osaka University Graduate, Osaka 565-0871 (JP); ICHIJO, Hidenori, c/o Graduate School Pharma., Bunkyo-ku Tokyo 111-0033 (JP)
(74) Representative: Owen, Deborah Jane
(86) International application number: PCT/JP2004/011124
(87) International publication number: WO 2005/009470

(57) **Abstract**

The present invention provides a drug for at least one of prevention and treatment of cardiac failure capable of suppressing cardiac depression and the onset of cardiac failure in ventricular remodeling, and a method for screening the drug. The drug for at least one of prevention and treatment of cardiac failure of the present invention contains a compound that inhibits a functional expression of ASK1 protein in a cardiomyocyte as an active ingredient, and a method for screening a drug for at least one of prevention and treatment of cardiac failure of the present invention includes selecting a medicinal component for at least one of prevention and treatment of cardiac failure from a drug candidate compound by using inhibition of a functional expression of ASK1 protein as an indication. As shown in FIG. 1, if ASK1 protein is removed, for example, the ventricle dilation can be attenuated in ventricular remodeling after myocardial infarction, pressure loading, or the like, which makes it possible to prevent and treat cardiac failure.

## Description

### Technical Field

The present invention relates to a remedy for cardiac failure containing ASK1 inhibitor as an active ingredient and a method for screening the same.

### Background Art

Cardiac failure is a syndrome presenting a variety of systemic subjective/objective signs due to a contraction dysfunction of the myocardium, and involves a threat to life. A patient suffering from cardiac failure has decreased exercise tolerance due to these conditions, is hospitalized repeatedly due to the exacerbation of cardiac failure, and finally reaches death. Thus, the prognosis of cardiac failure is very unsatisfactory. At present, as drug treatment, a diuretic, a digitalis preparation, a β-blocker, an angiotensin converting enzyme inhibitor, an angiotensin receptor inhibitor, and the like are used for the prevention and treatment of cardiac failure. Although the effects thereof have been demonstrated by a large-scale clinical test, they are small, which makes it necessary to depend upon a heart transplant in the case of serious chronic cardiac failure. Therefore, there is a demand for the development of a novel method for treating cardiac failure.

The cause of cardiac failure has not been clarified sufficiently. However, for example, the following clinical progress is known: ventricular remodeling (reconstruction) occurs after heart disease such as myocardial infarction, hypertension, and valvular heart disease, and then cardiac failure occurs. The ventricular remodeling is a change in geometry, mass, capacity, and function of the left ventricle responding to myocardial failure or a change in loading. The process of the above-mentioned remodeling is adaptable. However, in the case where the above-mentioned myocardial failure and loading are continuously abnormal, as in myocardial infarction, hypertension, and valvular heart disease, the process becomes maladaptable. Then, a sufficient amount of blood is not supplied to enlarged cardiomyocytes, leading to ischemia. This is considered to cause myocardial failure such as cardiac contraction failure, which results in a decrease in cardiac output, an organ circulation disorder, venous congestion, body fluid stagnation, and the like. Thus, the degree of the dilation of the left ventricle during remodeling is a strong indication regarding the state of disease and the mortality rate (see Patten, R.D., Udelson, J.E. & Konstam, M.A. "Ventricular remodeling and its prevention in the treatment of hear failure" (1998) Curr. Opin. Cardiol. 13, 162-7).

Regarding the mechanism to be a base for left ventricular remodeling after myocardial infarction, the involvement of apoptosis of myocytes is suggested in a human patient as well as an experimental model (see Olivetti, G., Quaini, F., Sala, R., Lagrasta, C., Corradi, D., Bonacina, E., Gambert, S.R., Cigola, E. & Anversa, P. "Acute Myocardial Infarction in Humans is Associated with Activation of Programmed Myocyte Cell Death in the Surviving Portion of the Heart" (1996) J. Mol. Cell. Cardiol. 28, 2005-2016, and Cheng, W., Kajstura, J., Nitahara, J. A., Li, B., Reiss, K., Liu, Y., Clark, W. A., Krajewski, S., Reed, J. C., Olivetti, G., et al. "Programmed Myocyte Cell Death Affects the Viable Myocardium after Infarction in Rats" (1996) Exp. Cell Res. 226, 316-327). Furthermore, it also is considered that the apoptosis of cardiomyocytes is an important point in transition between compensatory hypertrophy and cardiac failure even in cardiac failure occurring after the megalocardia responding to pressure loading (see Hirota, H., Chen, J., Betz, U.A., Rajewksy, K., Gu, Y., Ross, J., Jr., Muller, W. & Chien, K.R. "Loss of a gp130 Cardiac Muscle Cell Survival Pathway is a Critical Event in the Onset of Heart Failure During Biomechanicalstress" (1999) Cell 97, 189-98).

On the other hand, ASK1 (apoptosis signal-regulating kinase 1) protein is mitogen-activated protein kinase kinase kinase (MAPKKK) showing sensitivity to reactive enzyme species, which is a protein identified to activate a c-Jun N-terminal kinase (JNK) and a p38 MAP kinase (see JP 10(1998)-000093 A and Ichijo, H., Nishida, E., Irie, K., Dijike, P.T., Saitoh, M., Moriguchi, T., Takagi, M., Matumoto, K., Miyazono, K. & Gotoh, Y. "Induction of Apoptosis by ASK1, a Mammalian MAPKKK that Activates SAPK/JNK and p38 Signaling Pathways" (1997) Science 275, 90-94). The following have been reported: the overexpression of ASK1 protein of a wild type or a constitutively active form induces apoptosis in various cells (see Saitoh, M., Nishitoh, H., Fujii, M., Takeda, K., Tobiume, K., Sawada, Y, Kawabata, M., Miyazono, K. & Ichijo, H. (Mammalian Thioredoxin is a Direct Inhibitor of Apoptosis Signal-Regulating Kinase (ASK) 1" (1998) EmboJ. 17, 2596-606); and apoptosis induced by oxidative stress and a tumor necrosis factor is suppressed with ASK^{-/-} cells (see Tobiume, K., Matsuzawa, A., Takahashi, T., Nishitoh, H., Morita, K.-i., Takeda, K., Minowa, O., Miyazono, K., Noda, T. & Ichijo, H. "ASK1 is Required for Sustained Activations of JNK/p38 MAP Kinases and Apoptosis" (2001) EMBO Reports 2, 222-228). However, the relationship between cardiac failure and ASK1 has not been reported.

### Disclosure of Invention

### Problems to be Solved by the Invention

The object of the present invention is to provide a drug for at least one of the prevention and the treatment of cardiac failure, capable of suppressing cardiac depression and the onset of cardiac failure in the above-mentioned remodeling, a screening method thereof, a method for at least one of the prevention and the treatment of cardiac failure, and a method for diagnosing cardiac failure.

### Means for Solving the Problems

In order to achieve the above-mentioned object, the inventors of the present invention came up with an idea of reproducing left ventricular remodeling after myocardial infarction that is the cause of disease most related to cardiac failure clinically and left ventricular remodeling after pressure loading, using an ASK1 knockout mouse, and clarifying the molecular mechanism of the left ventricular remodeling, and have studied earnestly. Consequently, the inventors of the present invention found the following: even if ASK1 protein is removed from cardiomyocytes, morphological and histological defects do not occur in the heart at a basal level; the removal of ASK1 protein can suppress the dilation of the left ventricle involving a decrease in a progressive contraction function in left ventricular remodeling after myocardial infarction or pressure loading; the removal of ASK1 protein can suppress a progressive increase in the frequency of apoptosis in left ventricular remodeling after myocardial infarction or pressure loading; the activation of ASK1 protein induces apoptosis in cardiomyocytes; ASK1 protein is activated in the heart after myocardial infarction or pressure loading; and the like, thereby achieving the present invention.

More specifically, a drug for at least one of prevention and treatment of cardiac failure of the present invention contains a compound that inhibits a functional expression of ASK1 protein in cardiomyocytes as an active ingredient; a method for screening a drug for at least one of prevention and treatment of cardiac failure of the present invention includes selecting a medicinal component for at least one of prevention and treatment of cardiac failure from a drug candidate compound by using inhibition of a functional expression of ASK1 protein as an indication; and a method for diagnosing cardiac failure of the present invention includes measuring kinase activity or autophosphorylation of ASK1 protein in cardiomyocytes.

### Effects of the Invention

The drug for at least one of the prevention and the treatment of cardiac failure of the present invention can inhibit the functional expression of ASK1 protein, so that the drug can suppress, for example, a progressive decrease in a heart contraction function in the left ventricle of heart disease. Thus, for example, the drug can be used for preventing cardiac failure with respect to disease that may cause ventricular remodeling, such as myocardial infarction, hypertension, valvular heart disease, congenital heart disease, myocarditis, familial hypertrophic cardiomyopathy, and congestive cardiomyopathy. Furthermore, for example, the drug can be used for treating cardiac failure.

Furthermore, according to the screening method of the present invention, for example, it becomes possible to prepare a drug effective for the prevention or treatment of cardiac failure. Furthermore, according to the method for at least one of the prevention and the treatment of the present invention, for example, it becomes possible to prevent or treat cardiac failure effectively. Furthermore, according to the diagnostic method of the present invention, for example, it becomes possible to select effective diagnostic and treatment methods.

### Brief Description of Drawings

FIG. 1A shows exemplary transthoracic M-mode ultrasonic echocardiograms in an example of the present invention, and FIG. 1B shows exemplary graphs illustrating a change in parameters of echocardiography in an example of the present invention.
FIG. 2 shows exemplary photographs of heart sample sections in the example of the present invention.
FIG. 3A shows exemplary graphs illustrating the number of apoptosis cells in the example of the present invention, and FIG. 3B shows exemplary observations with triple staining microscopy in the example of the present invention.
FIG. 4A shows an exemplary graph illustrating cell survival rate in the example of the present invention, FIG. 4B shows exemplary observations with Hoechst dye microscopy in the example of the present invention, FIG. 4C shows another exemplary graph illustrating cell survival rate in the example of the present invention, and FIG. 4D shows other exemplary observations with Hoechst die microscopy in the example of the present invention.
FIG. 5A shows one exemplary measurement result of activation of ASK1 protein in the example of the present invention, and FIG. 5B shows one exemplary result obtained by measuring the phosphorylation of JNK protein and p38 protein in the example of the present invention.

### Description of the Invention

In the present invention, ASK1 protein is, for example, ASK1 protein of a mammal, preferably ASK1 protein of a human, more preferably an amino acid sequence of GenBank Database Registration Number D84476 or an amino acid sequence that is substantially the same as that of the amino acid sequence of GenBank Database Registration Number D84476, and further preferably ASK1 protein composed of an amino acid sequence that is the same or substantially the same as that of ASK1 protein of a patient to which the drug of the present invention is applied. Examples of substantially the same amino acid sequence include an amino sequence having a sequence homology of about 50% or more, preferably about 60% or more, more preferably about 70% or more, further preferably about 80% or more, particularly preferably about 90% or more, and most preferably 95% or more, and encoding a protein having activity of ASK1 protein.

The drug for at least one of the prevention and the treatment of cardiac failure of the present invention is not particularly limited, as long as it is a drug containing a compound that inhibits the functional expression of ASK1 protein in cardiomyocytes as an active ingredient, and examples of the drug include the following forms (1) to (8).
(1) As one embodiment of the present invention, the drug for at least one of the prevention and the treatment of cardiac failure of the present invention contains, as an active ingredient, a compound that suppresses apoptosis of cardiomyocytes induced by ASK1 protein.
(2) As another embodiment of the present invention, the drug for at least one of the prevention and the treatment of cardiac failure of the present invention contains, as an active ingredient, a compound that inhibits kinase activity of ASK1 protein in cardiomyocytes.
(3) As still another embodiment of the present invention, the drug for at least one of the prevention and the treatment of cardiac failure of the present invention contains, as an active ingredient, a compound that inhibits autophosphorylation of ASK1 protein in cardiomyocytes.
(4) As still another embodiment of the present invention, the drug for at least one of the prevention and the treatment of cardiac failure of the present invention contains, as an active ingredient, a compound that inhibits the translation of ASK1 mRNA in cardiomyocytes.
(5) As still another embodiment of the present invention, the drug for at least one of the prevention and the treatment of cardiac failure of the present invention contains, as an active ingredient, a compound that inhibits the transcription of ASK1 gene in cardiomyocytes.
(6) As still another embodiment of the present invention, the drug for at least one of the prevention and the treatment of cardiac failure of the present invention contains, as an active ingredient, a compound that inhibits a factor activating ASK1 protein in cardiomyocytes.
(7) As still another embodiment of the present invention, the drug for at least one of the prevention and the treatment of cardiac failure of the present invention contains, as an active ingredient, a compound that inhibits a factor activated by ASK1 protein in cardiomyocytes.
(8) As still another embodiment of the present invention, the drug for at least one of the prevention and the treatment of cardiac failure of the present invention contains, as an active ingredient, a compound that can suppress progressive cardiac depression in ventricular remodeling in cardiomyocytes.
   Examples of the above-mentioned compound that inhibits kinase activity of ASK1 protein or the above-mentioned compound that inhibits autophosphorylation of ASK1 protein include anti-ASK1 antibody specific to ASK1 protein. The anti-ASK1 antibody may be a polyclonal antibody, a monoclonal antibody, or a known antibody, or may be prepared newly. There is no particular limit to a method for preparing a polyclonal or monoclonal antibody, and a conventionally known method can be used. Furthermore, the anti-ASK1 antibody may be an antibody fragment. Examples of the antibody fragment include F(ab')2, Fab, Fab', and an Fv fragment. These fragments may be subjected to conventionally known various modifications. Furthermore, the above-mentioned anti-ASK1 antibody may be a chimera antibody.
   Furthermore, examples of the above-mentioned compound that inhibits kinase activity of ASK1 protein or the above-mentioned compound that inhibits autophosphorylation of ASK1 protein include a dominant negative variant of ASK1 protein. The ASK1 dominant negative variant is not particularly limited, as long as it can inhibit at least one of kinase activity, autophosphorylation activity, and complexation of ASK1 protein. Examples of the ASK1 dominant negative variant include ASK1(K709M), ASK1 (K709R), ASK1NT, ASK-ΔN(K709M), and ASK-ΔN(K709R). The K709M and K709R show that lysine (K) at the 709th position of ASK1 protein is replaced by methionine (M) and arginine (R). The above-mentioned ASK1NT represents a protein composed of amino acid residues 1 to 648 that correspond to an N-terminal region of ASK1 protein, and the above-mentioned ASK-△N represents protein composed of amino acid residues 649 to 1375 that correspond to a C-terminal region of ASK1 protein.
   Furthermore, an example of the above-mentioned compound that inhibits kinase activity of ASK1 protein or the above-mentioned compound that inhibits autophosphorylation of ASK1 protein includes thioredoxin.
   An example of the above-mentioned compound that inhibits the translation of ASK1 mRNA includes an antisense polynucleotide. The antisense polynucleotide is not particularly limited as long as it can be paired complementarily with ASK1 mRNA, and can suppress the expression of ASK1 protein. Examples of the antisense polynucleotide include antisense DNA, antisense RNA, antisense DNA/RNA chimera, and a derivative thereof. It is preferable that the sequence of the antisense polynucleotide is the same or substantially the same as a complementary sequence of ASK1 mRNA. In the present invention, an ASK1 mRNA sequence is, for example, an ASK1 mRNA sequence of a mammal, preferably an ASK1 mRNA sequence of a human, more preferably a sequence of GenBank Database Registration Number D84476, and more preferably a sequence that is the same or substantially the same as that of an ASK1 mRNA sequence of a patient to which the drug of the present invention is applied. Examples of substantially the same amino acid sequence include a sequence having a sequence homology of about 50% or more, preferably about 60% or more, more preferably about 70% or more, further preferably about 80% or more, particularly preferably about 90% or more, and most preferably 95% or more, and encoding an amino acid sequence that is the same or substantially the same as that of ASK1 protein.
   Furthermore, an example of the above-mentioned compound that inhibits the translation of ASK1 mRNA includes RNA for RNA interference. Examples of the above-mentioned RNA for RNA interference include single-stranded, double-stranded, or triple-stranded RNA having the same sequence as the above-mentioned ASK1 mRNA sequence. The RNA for RNA interference is preferably double-stranded RNA having the same sequence as the ASK1 mRNA sequence, and more preferably double-stranded RNA having the same sequence as the above-mentioned ASK1 mRNA composed of a RNA fragment of 19 to 25 nucleotides.
   Examples of the above-mentioned activating factor of ASK1 protein include Daxx, and TRAF2, Ca²⁺/calmodulin-dependent protein kinase II; CaM Kinase II. Examples of the above-mentioned factor activated by ASK1 protein include MKK3, MKK4, MKK6, MKK7, JNK, and p38 MAPK. Among them, in one embodiment of the drug of the present invention, MKK4, MKK7, and JNK are preferable as the above-mentioned factor activated by ASK1 protein that is inhibited by an active ingredient.
   The compound contained as an active ingredient in the drug for at least one of the prevention and the treatment of cardiac failure of the present invention contains, for example, a low-molecular inorganic compound, a low-molecular organic compound, DNA, RNA, a peptide, protein, lipid, sugar, a derivative thereof, or a salt thereof. In the case where the above-mentioned compound of the active ingredient is, for example, DNA, RNA, a peptide, and protein, a nucleotide sequence encoding them can be used. Thus, an example of further one embodiment of the drug for at least one of the prevention and the treatment of cardiac failure of the present invention includes the following form (9).
(9) As still another embodiment of the present invention, the drug for at least one of the prevention and the treatment of cardiac failure of the present invention is a vector containing a nucleotide sequence encoding a compound that is an active ingredient, wherein the nucleotide sequence contains a vector operatively connected to a regulatory sequence required for expressing the nucleotide sequence.

The above-mentioned vector is not particularly limited, as long as it is capable of inserting the above-mentioned nucleotide sequence in a patient's body, preferably in cardiomyocytes. For example, single-stranded, double-stranded, cyclic, or supercoiled DNA molecules and RNA molecules can be used. Specific examples of the vector include virus vectors such as a retrovirus vector, an adenovirus vector, an adenovirus associated virus vector, and non-virus vectors such as pCAGGS (Gene 108, 193-200 (1991)), pBK-CMV, pcDNA3, and pZeoSV (produced by Invitrogen Corporation). The regulatory sequence is a sequence required for expressing the nucleotide sequence to which it is operatively connected in patient's cells, preferably cardiomyocytes. Examples of the regulatory sequence suitable for eucaryotic cells include a promoter, a polyadenylated signal, and an enhancer. Being operatively connected refers to each constituent element being aligned so as to achieve its function.

The drug for at least one of the prevention and the treatment of cardiac failure of the present invention can be used, for example, in a mammal, preferably a human. Furthermore, the drug of the present invention is applicable to disease such as cardiac failure, myocardial infarction, hypertension, valvular heart disease, valvular heart disease, congenital heart disease, myocarditis, familial hypertrophic cardiomyopathy, and congestive cardiomyopathy.

Examples of an administration route of the drug for at least one of the prevention and the treatment of cardiac failure of the present invention include oral administration and parenteral administration. Examples of the parenteral administration include intraoral administration, intratracheal administration, intrarectal administration, subcutaneous administration, intramuscular administration, and intravenous administration. Furthermore, examples of the administration form include an oral administration agent, a nasal agent, a percutaneous agent, a rectal administration agent (suppository), a sublingual agent, a transvaginal agent, an injection (transvenous agent, transartery agent, subcutaneous agent, intracutaneous agent), and a drip, depending upon the administration route. Furthermore, examples of the oral administration agent include a tablet, a pill, a powdered medicine, a powdered drug, a granule, a capsule, a solution, a suspension, an emulsion, and a syrup. Examples of the percutaneous agent include a liquid agent such as lotion and a semi-solid agent such as creme and ointment. The administration route and the administration form of the drug of the present invention are not limited to the above, and it is desirable to use the one that is most effective for at least one of the prevention and the treatment.

In the case where the drug for at least one of the prevention and the treatment of cardiac failure of the present invention takes a form including the above-mentioned vector, i.e., an agent form of a so-called gene therapeutic agent, it is preferable that the drug is introduced in a patient's body, preferably in cardiomyocytes, depending upon the vector.

In the case where the above-mentioned vector is a virus vector, examples of a method for administering the vector include an *in vivo* method and an *ex vivo* method. According to the *in vivo* method, the drug is administered through an appropriate administration route in accordance with an intended disease, a target organ, or the like. For example, the drug may be administered in the vein, the artery, the coronary artery, subcutaneously, intracutaneously, or in the myocardium, or may be locally administered directly to a site recognized to be a lesion. On the other hand, in the case where the vector is a non-virus vector, examples of a method for administering the vector include an introduction method through a internal capsule-type liposome or a static liposome, an HVJ-liposome method, a modified HVJ-liposome method, a receptor interstitial introduction method, a method for introducing the vector together with a carrier of metal particles with a particle gun, a direct introduction method for naked-DNA, and an introduction method using a positively charged polymer.

The method for screening a drug for at least one of the prevention and the treatment of cardiac failure of the present invention is not particularly limited, as long as it includes the process of selecting a medicinal component for at least one of the prevention and the treatment of cardiac failure from drug candidate compounds by using the inhibition of the functional expression of ASK1 protein as an indication. Examples of the screening method include the following forms (1) to (8).
(1) As one embodiment of the present invention, the method for screening a drug for at least one of the prevention and the treatment of cardiac failure of the present invention includes the process of selecting a medicinal component for at least one of the prevention and the treatment of cardiac failure from drug candidate compounds by using the suppression of apoptosis induced by ASK1 protein as an indication.
(2) As another embodiment of the present invention, the method for screening a drug for at least one of the prevention and the treatment of cardiac failure of the present invention includes the process of selecting a medicinal component for at least one of the prevention and the treatment of cardiac failure from drug candidate compounds by using the inhibition of kinase activity of ASK1 protein as an indication.
(3) As still another embodiment of the present invention, the method for screening a drug for at least one of the prevention and the treatment of cardiac failure of the present invention includes the process of selecting a medicinal component for at least one of the prevention and the treatment of cardiac failure from drug candidate compounds by using the inhibition of autophosphorylation of ASK1 protein as an indication.
(4) As still another embodiment of the present invention, the method for screening a drug for at least one of the prevention and the treatment of cardiac failure of the present invention includes the process of selecting a medicinal component for at least one of the prevention and the treatment of cardiac failure from drug candidate compounds by using the inhibition of translation of ASK1 mRNA as an indication.
(5) As still another embodiment of the present invention, the method for screening a drug for at least one of the prevention and the treatment of cardiac failure of the present invention includes the process of selecting a medicinal component for at least one of the prevention and the treatment of cardiac failure from drug candidate compounds by using the inhibition of transcription of ASK1 gene as an indication.
(6) As still another embodiment of the present invention, the method for screening a drug for at least one of the prevention and the treatment of cardiac failure of the present invention includes the process of selecting a medicinal component for at least one of the prevention and the treatment of cardiac failure from drug candidate compounds by using the inhibition of a factor activating ASK1 protein as an indication.
(7) As still another embodiment of the present invention, the method for screening a drug for at least one of the prevention and the treatment of cardiac failure of the present invention includes the process of selecting a medicinal component for at least one of the prevention and the treatment of cardiac failure from drug candidate compounds by using the inhibition of a factor activated by ASK1 protein as an indication.
(8) As still another embodiment of the present invention, the method for screening a drug for at least one of the prevention and the treatment of cardiac failure of the present invention includes the process of selecting a medicinal component for at least one of the prevention and the treatment of cardiac failure from drug candidate compounds by using the suppression of progressive cardiac depression of ventricular remodeling as an indication.

The above-mentioned screening method can be performed using a conventionally known method *in silico, in vitro,* and *in vivo.* Furthermore, the medicinal component selected in the above-mentioned process of selecting a medicinal component may be used directly as an active ingredient of the drug for at least one of the prevention and the treatment of cardiac failure of the present invention. In the case where the above-mentioned medicinal component is, for example, a polynucleotide or a polypeptide, the drug may be used in a form of the above-mentioned gene therapeutic agent containing a gene encoding a polynucleotide or a polypeptide. Examples of the drug candidate compounds include a low-molecular inorganic compound, a low-molecular organic compound, DNA, RNA, a peptide, protein, lipid, sugar, a derivative thereof, and a salt thereof.

Specific examples of the above-mentioned screening method by using the suppression of apoptosis as an indication include a screening method for administering a drug candidate compound to cells that express ASK1 protein (e.g., ASK-△N; AKS1 protein lacking amino acids 1 to 648 in an N-terminal region) having constitutive activity or a transgenic mouse that expresses the above-mentioned constitutively active mutant ASK-ΔN in the heart.

A specific example of the above-mentioned screening method by using the inhibition of kinase activity as an indication includes a screening method for administering an ASK1 activating material to cells in the presence of a drug candidate compound, collecting ASK1 protein immunoprecipitated with an anti-ASK1 antibody from the cells, and measuring kinase activity of ASK1 protein, thereby selecting a drug capable of suppressing kinase activity of ASK1 protein.

A specific example of the above-mentioned screening method by using inhibition of autophosphorylation as an indication includes a screening method for administering ASK1 activating material to cells in the presence of a drug candidate compound, and performing Western blot using an anti-phosphorylated ASK1 antibody with the cells being a sample or performing Western blot using an anti-phosphorylated ASK1 antibody with respect to ASK1 protein immunoprecipitated with an anti-ASK1 antibody from the cells, thereby selecting a drug capable of suppressing autophosphorylation of ASK1 protein.

A specific example of the above-mentioned screening method by using inhibition of translation of ASK1 mRNA includes a screening method for extracting protein from cells or the animal heart in the presence of a drug candidate compound, and evaluating the expression of ASK1 protein, thereby selecting a drug inhibiting the translation of ASK1 mRNA.

A specific example of the above-mentioned screening method by using the inhibition of transcription of ASK1 gene includes a screening method for isolating mRNA from cells or the animal heart in the presence of a drug candidate compound, and evaluating the expression of ASK1 mRNA, thereby selecting a drug inhibiting the transcription of ASK1 gene.

A specific example of the above-mentioned screening method by using the inhibition of a factor activated by ASK1 protein as an indication includes a screening method for administering a drug candidate compound to cells that express a constant activator ASK-ΔN or a transgenic mouse that expresses the constant activator ASK-ΔN in the heart, and evaluating the activity of, for example, MKK3, MKK4, MKK6, MKK7, JNK, p38MAPK, preferably MKK4, MKK7, JNK, thereby selecting a drug inhibiting a factor activated by ASK1 protein. The method for evaluating activity is not particularly limited, and for example, the activity can be evaluated by detecting the phosphorylation of a factor with an antibody or the like.

These screening methods are examples of the screening method of the present invention, and the screening method of the present invention is not limited thereto. Furthermore, in the above-mentioned screening methods, the handling of an experimental animal, cells, protein, nucleic acid, and the like is not particularly limited, and can be performed by a conventionally known method.

A method for at least one of the prevention and the treatment of cardiac failure of the present invention is not particularly limited, as long as it includes inhibiting the functional expression of ASK1 protein in cardiomyocytes, and examples of the method include the following forms (1) to (6).
(1) As one embodiment of the present invention, the method for at least one of the prevention and the treatment of cardiac failure of the present invention is a method for at least one of the prevention and the treatment, which includes suppressing apoptosis induced by ASK1 protein.
(2) As another embodiment of the present invention, the method for at least one of the prevention and the treatment of cardiac failure of the present invention is a method for at least one of the prevention and the treatment, which includes inhibiting kinase activity of ASK1 protein in cardiomyocytes.
(3) As still another embodiment of the present invention, the method for at least one of the prevention and the treatment of cardiac failure of the present invention is a method for at least one of the prevention and the treatment, which includes inhibiting autophosphorylation of ASK1 protein in cardiomyocytes.
(4) As still another embodiment of the present invention, the method for at least one of the prevention and the treatment of cardiac failure of the present invention is a method for at least one of the prevention and the treatment, which includes inhibiting transcription translation activity of ASK1 gene in cardiomyocytes.
(5) As still another embodiment of the present invention, the method for at least one of the prevention and the treatment of cardiac failure of the present invention is a method for at least one of the prevention and the treatment, which includes at least one of an ASK1 activating factor and a factor activated by ASK1 protein.
(6) As still another embodiment of the present invention, the method for at least one of the prevention and the treatment of cardiac failure of the present invention is a method for at least one of the prevention and the treatment, which includes administering a pharmaceutically acceptable effective amount of drug for at least one of the prevention and the treatment of cardiac failure of the present invention.

Regarding an administration method and an administration form of a drug used for the above-mentioned at least one of the prevention and the treatment, as described above, the most effective ones for at least one of the prevention and the treatment can be selected.

Furthermore, a method for diagnosing cardiac failure of the present invention includes measuring kinase activity or autophosphorylation ability of ASK1 protein in cardiomyocytes. By measuring the above-mentioned kinase activity and autophosphorylation ability, for example, the seriousness and qualitative state of cardiac failure and heart disease that may develop cardiac failure can be diagnosed. This enables the optimum prevention/treatment method for cardiac failure to be selected. Examples of the heart disease that may develop cardiac failure include myocardial infarction, hypertension, valvular heart disease, a congenital heart disease, myocarditis, familial hypertrophic cardiomyopathy, and congestive cardiomyopathy.

Hereinafter, the present invention will be described in more detail by way of a specific example. The present invention is not limited to the following example. The following procedure was used for carrying out the example.

### Example

### (ASK1 knockout mouse and experimental model)

As ASK1 knockout mice, the ones in the F6 generation of C57B16/J background, which have already been reported, were used (Tobiume, K. et al., (2001) EMBO Reports 2, 222-228), and as control wild type mice (WT mouse), C57B16/J mice (produced by Japan SLC, Inc.) as old as those in the F6 generation were used. Surgical treatments for a myocardial infarction model and a thoracic transverse aortic constriction (TAC) model were performed using 10-week old mice. The above-mentioned myocardial infarction was caused by ligation of the left coronary artery as described in the literature (Otsu, K. et al., (2003) Biochem. Biophys. Res. Commun. 302, 56-60), and furthermore, the TAC treatment was performed as described in the literature (Date, M.O. et al., (2002) J. Am. Coll. Cardiol. 39, 907-12).

### (Echocardiography)

Echocardiography was carried out using ultra-sonography (SONOS-5500 equipped with a linear converter of 15-MHz, produced by Philips Medical Systems) by anesthetizing mice with 2.5% avertin (8 µl/g). The heart was imaged with a two-dimensional parasternal short-axis view, and an M-mode echocardiogram of the midvetricle was recorded at a level of the papillary muscle. A heartbeat, anterior and posterior wall thicknesses, a left ventricular internal diameter in end diastole (LVIDd), and a left ventricular internal diameter in end systole (LUIDs) were obtained from the above-mentioned M-mode image.

### (In vivo evaluation of a cardiac function by cardiac catheterization)

For the purpose of cardiac catheterization, 10-week old mice were injected intraperitoneally with a mixture of ketamine (50-100 mg/kg) and xylazine (3-6 mg/kg), thereby being anesthetized. Then, the right carotid artery of each mouse was separated, and a cannula was inserted in the right carotid artery together with a 1.4 French Millar catheter connected to an amplifier (TCP-500, Millar Inc.), as described in the literature (Nakayama, H. et al., (2002) FASEB J., 0.2-0474fje). After the catheter was inserted in the right carotid artery, the catheter was allowed to proceed from the aorta to the left ventricle. The left ventricle was digitized, and processed by a computer system.

### (Histological analysis)

A heart sample was arrested in diastole, immediately fixed with a 3.7% formalin buffer, and embedded in a paraffin to obtain a section sample with a thickness of 3 µm. Hematoxylin and eosin (HE) staining or Masson-trichrome staining was performed on serial sections.

### (In vitro kinase assay)

The kinase activity of ASK1 protein *in vitro* was measured by immune complex kinase assay as described previously (Ichijo, H. et al., (1997) Science 275, 90-4). The immunoprecipitation of endogenous ASK1 was performed with respect to 500 µg of a myocardium extract as reported (Saitoh, M. et al., (1998) Embo J. 17, 2596-606).

### (Evaluation of apoptosis)

The evaluation of apoptosis was performed by terminal deoxyribonucleotidyl transferase biotin-dUTP nick end labeling (TUNEL) assay. This assay was performed with respect to a heart section embedded in a paraffin in accordance with an instruction manual of a producer, using an *in-situ* apoptosis detection kit (produced by Takara). The number of TUNEL positive nuclei was counted by checking the entire section with a ×40 objective. Some samples were subjected to triple staining using propidium iodide (produced by Vector Laboratories Inc.), TUNEL, and an anti-alpha-sarcomere (striated fiber) actin antibody.

### (Primary culture of neonatal rat ventricular myocytes and survival assay)

Ventricular myocytes of rats obtained from 1 to 2-day old Wistar rats were prepared and cultured as described in the literature (Hirotani, S. et al., (2002) Circulation 105, 509-15). An adenovirus vector that expresses a constitutively active form of ASK1 (AdASK-△N) or β-galactosidase (AdLacZ) was as previously described (Saitoh, M. et al., (1998) Embo J. 17, 2596-606). The cardiomyocytes were infected with the recombinant adenovirus vector for one hour at a multiplicity of infection of 100 plaque forming unit per cell. After this, the cells were cultured further for 24 hours or 48 hours. The relative cell number with the value on the 0th day being 1 was measured three times using Cell Counting Kit-8 (produced by Dojindo) based on 3-[4,5-dimethylthiazol-2-yl]-2,5-diphenyltetrazolium bromide (MTT) assay. The cell staining by Hoechst 33258 was performed by incubating the cells in a 160 µM solution.

### (Assay of MARK phosphorylation)

The activation of JNK and p38 was evaluated by subjecting a myocardium extract to Western blot using an anti-phosphorylated JNK antibody or an anti-phosphorylated p38 antibody.

The results of the experiments were shown as an average ± standard error (SEM). Paired data was evaluated by a Student's test. Analysis of variance (ANOVA) in a one-dimensional arrangement and the Bonferroni's post hoc test or repeated measures ANOVA were used for multiple comparisons. The value of P < 0.05 was considered to be significant statistically

### (Characterization of the heart in an ASK1 knockout mouse)

The hearts of ASK1 knockout mice were compared with the hearts of control wild type (WT) mice. The ASK1 knockout mice were born at an expected frequency of Mendelian inheritance, and were not apparently distinguished from the control WT mice. Furthermore, the ASK1 knockout mice were compared with the control WT mice in terms of the weight of the body, the left ventricle, the right ventricle, and the atrium. Table 1 shows the results. As shown in the following Table 1, no significant difference was recognized therebetween. The hearts of the ASK1 knockout mice did not show any signs of morphologic injuries, and even in the above-mentioned histological analysis of the hearts, the disturbance of myofibril and necrosis, or ventricular fibrosis were not recognized. Furthermore, the myocyte cross-sectional areas of ASK1 knockout mice were not different from those of the control WT mice (338 ± 12 µm² in the ASK knockout mice, and 328 ± 12 µm² in the control WT).

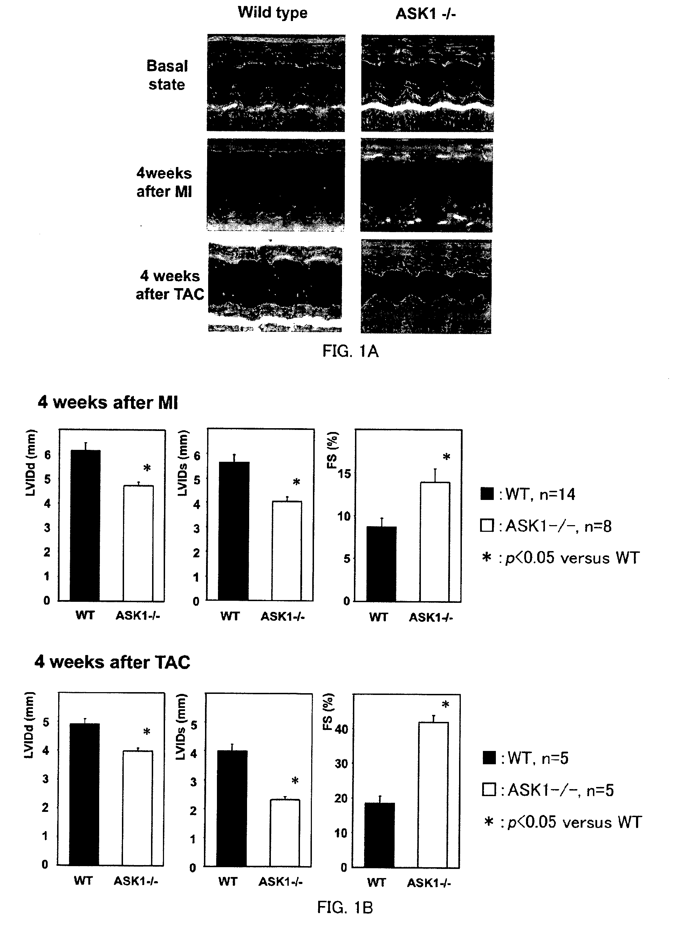

Next, in order to determine whether or not the knockout of ASK1 influences a cardiac function, the performance of the hearts of the ASK1 knockout mice was evaluated by echocardiography and cardiac catheterization in 10-week old mice. The results of the evaluation of a left ventricular internal diameter in end diastole, a left ventricular internal diameter in end systole, a left ventricular fractional shortening, a diastolic interventricular septum wall thickness, a diastolic left ventricular posterior wall thickness, and a heartbeat. Furthermore, an upper section of FIG. 1A shows exemplary transthoracic M-mode ultrasonic echocardiograms. In FIG. 1A, a right column shows the ASK1 knockout mouse, and a left column shows the control WT mouse. As shown in the following Table 1 and FIG. 1A, in the above-mentioned evaluation items, no significant difference was recognized between the ASK1 knockout mice and the control WT mice. The following Table 1 shows hemodynamic data regarding a left ventricular pressure in systole, a left ventricular pressure in end diastole, a maximum first-derivative of a left ventricular pressure, a minimum first-derivative of a left ventricular pressure, and a heartbeat. As shown in the following Table 1, even in the hemodynamic data, no significant difference was recognized between the ASK1 knockout mice and the control WT mice. The above findings suggest that the ASK1 knockout mouse has an entire configuration and function of the normal heart.

**(Table 1) Physiological parameters at a basal level, and analysis of a size and a function of the heart in vivo by cardiac catheterization and echocardiography.**

| Morphological Measurement Baseline | ASK1-/- (n = 5) | WT (n = 5) | |
|---|---|---|---|
| Body weight (g) | 23.0 ± 0.8 | 23.6 ± 0.2 | n.s. |
| Heart weight (mg) | 106.8 ± 3.8 | 106.0 ± 2.2 | n.s. |
| Left ventricle weight (mg) | 75.5 ± 2.0 | 74.1 ± 1.6 | n.s. |
| Right ventricle weight (mg) | 20.4 ± 2.0 | 18.4 ± 0.7 | n.s. |
| Atrium weight (mg) | 8.7 ± 1.3 | 10.1 ± 0.4 | n.s. |
| Tibia length (mm) | 16.8 ± 0.1 | 17.2 ± 0.2 | n.s. |
| Left ventricle weight/Body weight (mg/g) | 3.29 ± 0.07 | 3.14 ± 0.06 | n.s. |
| Left ventricle weight/Tibia length (mg/mm) | 4.48 ± 0.11 | 4.30 ± 0.11 | n.s. |
| Left ventricular pressure in systole (mmHg) | 86.0 ± 2.0 | 83.2 ± 3.0 | n.s. |
| Left ventricular pressure in end diastole (mmHg) | 0.9 ± 0.9 | 0.5 ± 0.3 | n.s. |
| max dp/dt (mmHg/sec) | 6600 ± 270 | 6320 ± 490 | n.s. |
| min dp/dt (mmHg/sec) | -5300 ± 440 | -4900 ± 360 | n.s. |
| Heartbeat (beats/min) | 413 ± 28 | 393 ± 28 | n.s. |
| | | | |

| Echocardiography baseline | ASK-/- (n = 10) | WT (n = 10) | |
|---|---|---|---|
| Left ventricular internal diameter in end diastole (mm) | 3.84 ± 0.08 | 3.92 ± 0.06 | n.s. |
| Left ventricular internal diameter in end systole (mm) | 2.33 ± 0.06 | 2.37 ± 0.06 | n.s. |
| Left ventricular fractional shortening (%) | 39.3 ± 0.8 | 39.5 ± 0.7 | n.s. |
| Diastolic interventricular septum wall thickness (mm) | 0.68 ± 0.03 | 0.70 ± 0.02 | n.s. |
| Diastolic left ventricular posterior wall thickness (mm) | 0.62 ± 0.04 | 0.65 ± 0.03 | n.s. |
| Heartbeat (beats/min) | 528 ±14 | 531 ± 15 | n.s. |

| | | | |
|---|---|---|---|
| In the above Table 1, ASK-/- represents ASK1 knockout; n represents the number of samples; n.s. shows that no significant difference is recognized; and max dp/dt and min dp/dt represent a maximum change ratio of a pressure occurring in systole and in diastole respectively. The data is represented by an average ± standard deviation. | | | |

### (Cardiac function and anatomical configuration of a ventricle after myocardial infarction (MI) and TAC)

Each left coronary artery of an ASK1 knockout mouse and a control WT mouse was subjected to ligation to cause myocardial infarction. In myocardial infarction, remodeling occurs first along with side-side slippage between side surfaces of myocytes. Consequently, the infarct expands, and the myocardium in a non-infarct region away from an infarct region is enlarged in response to volume loading and a neurohumoral signal. In an initial stage, although this is useful for reducing the stress of a wall, finally, the left ventricle dilates, and the left ventricle wall becomes thin, with the result that a contraction function decreases. An early operation mortality rate (within 24 hours) after the above-mentioned surgical treatment was zero in both the ASK1 knockout mice and the control WT mice, and no significant difference was recognized therebetween for a mortality rate within 7 days (20% in the ASK1 knockout mice and 18% in the control WT mice). In all the examples of dead mice, excess bleeding filling the periphery of the heart or the thoracic cavity occurred, and hence the rupture of the left ventricle was recognized to be the cause of death. In a period from one week to four weeks after the above-mentioned surgical treatment, mice did not die. The physiological influence *in vivo* of the ASK1 knockout in the left ventricular remodeling after myocardial infarction was evaluated. For the purpose of the evaluation, echocardiography was performed continuously before the surgical treatment, two weeks after the treatment, and four weeks after the treatment. The following Table 2 shows the results, and a middle section of FIG. 1A shows exemplary transthoracic M-mode ultrasonic echocardiograms. In FIG. 1A, a right column shows the ASK1 knockout mouse (ASK^{-/-}), and a left column shows the control WT mouse. Furthermore, an upper section of FIG. 1B shows one example obtained by comparing a left ventricular internal diameter in end diastole (LVIDd), a left ventricular internal diameter in end systole (LVIDs), and a left ventricular fractional shortening (FS) four weeks after the above treatment. As shown in FIG. 1B and the following Table 2, the left ventricular internal diameter in end diastole and the left ventricular internal diameter in end systole after the treatment both increased; however, the degree of increase was significantly larger in the control WT mice than in the ASK1 knockout mice (ASK^{-/-}). Furthermore, the left ventricular fractional shortening of the ASK1 knockout mice (ASK^{-/-}) and that of the control WT mice decreased, and the degree of the decrease was significantly larger in the control WT mice than in the ASK1 knockout mice. In the ASK1 knockout mice and the control WT mice subjected to a sham operation (the left coronary artery was not ligated), no remarkable change was recognized in any of the left ventricular internal diameter in end diastole, the left ventricular internal diameter in end systole, and the left ventricular fractional shortening. The lung weight of the control WT mice four weeks after the ligation of the left coronary artery, and the weight ratio between the lung and the body (lung/body ratio) was significantly larger in the control WT mice than in the ASK1 knockout mice (lung weight was 153.3 ± 3.3 mg in the ASK1 knockout mice and 204.5 ± 15.3 mg in the control WT). Furthermore, the lung/body ratio was 5.63 ± 0.24 × 10³ in the ASK1 knockout mice, and 7.65 ± 0.53 × 10⁻³ in the control WT mice).

**(Table 2) Physiological parameters after myocardial infarction, and an analysis of a size and a function of the heart in vivo by echocardiography**

| ASK1 knockout (n = 14) | Basal level | After two weeks | After four weeks |
|---|---|---|---|
| Body weight (g) | 24.6 ± 1.9 | 25.4 ± 1.0 | 27.4 ± 2.0* |
| Left ventricular internal diameter in end diastole (mm) | 3.64 ± 0.09 | 4.33 ± 0.09 * | 4.72 ± 0.16 *† |
| Left ventricular internal diameter in end systole (mm) | 2.28 ± 0.06 | 3.78 ± 0.07* | 4.05 ± 0.19 *† |
| Left ventricular fractional shortening (%) | 39.1 ± 1.7 | 12.8 ± 0.4 * | 13.9 ± 1.6 *† |
| Diastolic interventricular septum wall thickness (mm) | 0.68 ± 0.03 | 0.34 ± 0.04 | 0.37 ± 0.05 * |
| Diastolic left ventricular posterior wall thickness (mm) | 0.62 ± 0.03 | 0.73 ± 0.03 | 0.77 ± 0.03 † |
| Diastolic blood pressure (mmHg) | 107 ± 2.5 | 109 ± 3.1 | 108 ± 3.8 |
| Heartbeat (beats/min) | 625 ± 20 | 615 ± 10 | 566 ± 14 |
| | | | |

| Control WT (n = 8) | Basal level | After two weeks | After four weeks |
|---|---|---|---|
| Body weight (g) | 23.3 ± 0.8 | 24.5 ± 1.3 | 26.5 ± 2.3 * |
| Left ventricular internal diameter in end diastole (mm) | 3,75 ± 0.06 | 5.72 ± 0.24 * | 6.17 ± 0.3 * |
| Left ventricular internal diameter in end systole (mm) | 2,37 ± 0.15 | 5.23 ± 0.25 * | 5.65 ± 0.3 * |
| Left ventricular fractional shortening (%) | 38.8 ± 1.0 | 8.8 ±1.0 * | 8.7 ± 1.0 * |
| Diastolic interventricular septum wall thickness (mm) | 0,73 ± 0.02 | 0.33 ± 0.02 * | 0.31 ± 0.01 * |
| Diastolic left ventricular posterior wall thickness (mm) | 0.65 ± 0.06 | 0.48 ± 0.06 * | 0.55 ± 0.05 * |
| Systolic blood pressure (mmHg) | 104 ± 4.8 | 104 ± 3.9 | 106 ± 4.0 |
| Heartbeat (beats/min) | 604 ± 5 | 613 ± 4 | 576 ± 6 |

| | | | |
|---|---|---|---|
| In the above Table 2, n represents the number of samples, and the data is represented by an average ± standard deviation; * P < 0.05 versus the same genotype mice at a basal level; and † P < 0.05 versus the control WT mice four weeks after the surgical treatment. | | | |

Next, the thoracic transverse aorta of the ASK1 knockout mouse and the control WT mouse were subjected to banding (TAC) to cause pressure loading. In response to the pressure loading, the heart activates an adaptable physiological response to be enlarged, thereby reducing the stress of a wall. It is known that even a mouse subjected to TAC shows hyperfunctional hypertrophy without showing any signs of cardiac failure at a time after one week. However, the exposure of long-term or excess mechanical stress results in the dilation of the ventricle and the atrium, and abnormal cardiac function. The physiological influence *in vivo* of the ASK1 knockout mouse in the left ventricular remodeling after pressure loading by the TAC treatment was evaluated. For the purpose of the evaluation, echocardiography was performed continuously before the surgical treatment, two weeks after the treatment, and four weeks after the treatment. The following Table 3 shows the results, and a lower section of FIG. 1A shows exemplary transthoracic M-mode ultrasonic echocardiograms. In FIG. 1A, a right column shows the ASK1 knockout mouse, and a left column shows the control WT mouse. Furthermore, a lower section of FIG. 1B shows one example obtained by comparing a left ventricular internal diameter in end diastole (LVIDd), a left ventricular internal diameter in end systole (LVIDs), and a left ventricular fractional shortening (FS) four weeks after the treatment. As shown in FIG. 1B and the following Table 3, one week after the TAC treatment, the heart weight and the weight ratio between the heart and the body (heart/body weight ratio) increased to the same degree both in the ASK1 knockout mice and the control WT mice. Even in a myocyte cross-sectional area at that time, no difference was recognized (443 ± 18 µm² in the ASK1 knockout mice, and 445 ± 15 µm² in the control WT). Furthermore, four weeks after the treatment, the left ventricular internal diameter in end diastole of the control WT mice increased more remarkably, compared with those of the ASK1 knockout mice and the mice subjected to a sham operation (not subjected to TAC treatment). The left ventricular fractional shortening also decreased more remarkably in the control WT mice, compared with those of the ASK1 knockout mice and the mice subjected to a sham operation. The lung weight and the lung/body weight ratio four weeks after the TAC treatment increased remarkably in the control WT mice, and did not increase in the ASK1 knockout mice.

**(Table 3) Physiological parameters after pressure loading by TAC, and an analysis of a size and a function of the heart in vivo by echocardiography**

| ASK1 knockout | Four weeks after sham operation (n=4) | One week after TAC (n = 5) TAC (n = 5) | Four weeks after TAC (n = 5) TAC (n = 5) |
|---|---|---|---|
| Body weight (g) | 30.4 ± 0.4 | 27.1 ± 0.8 | 29.9 ± 0.6 ‡ |
| Heart weight (mg) | 146 ± 3 | 193 ± 13 | 218 ± 8 * |
| Lung weight (mg) | 152 ± 4 | n.d. | 162 ± 4 † |
| Liver weight (mg) | 1470 ± 50 | n.d. | 1310 ± 50 |
| Heart/body weight ratio | 4.8 ± 0.1 | 7.1 ± 0.5 | 7.3 ± 0.2 *† |
| Lung/body weight ratio | 5.0 ± 0.1 | n.d. | 5.4 ± 0.1 † |
| Liver/body weight ratio | 49 ± 1 | n.d. | 44 ± 1 |
| Left ventricular internal diameter in end diastole (mm) | 3.63 ± 0.10 | 3.76 ± 0.09 | 3.98 ± 0.11 † |
| Left ventricular internal diameter in end systole (mm) | 2.09 ± 0.04 | 2.24 ± 0.07 | 2.32 ± 0.11 † |
| Left ventricular fractional shortening(%) | 42.4 ± 2.1 | 40.5 ± 1.1 | 41.9 ± 2.1 † |
| Diastolic ventricular septum wall thickness (mm) | 0.78 ± 0.03 | 1.00 ± 0.07 | 1.1 ± 0.02 *† |
| Diastolic left ventricular posterior wall thickness (mm) | 0.78 ± 0.04 | 0.91 ± 0.03 | 0.85 ± 0.03 |
| Heartbeat (beats/min) | 554 ± 16 | 548 ± 24 | 563 ± 26 |
| | | | |

| Control WT | Fourweeksafter Four weeks after sham operation (n = 3) | One week after TAC (n = 4) | Four weeks after TAC (n = 4) |
|---|---|---|---|
| Body weight (g) | 27.7 ± 0.6 | 26.2 ± 0.2 | 28.3 ± 0.9 |
| Heart weight (mg) | 137 ± 10 | 182 ± 9 | 253 ± 17 *† |
| Lung weight (mg) | 143 ± 7 | n.d. | 317 ± 41 * |
| Liver weight (mg) | 1360 ± 90 | n.d. | 1350 ± 140 |
| Heart/body weight ratio | 5.0 ± 0.3 | 6.9 ± 0.3 | 8.9 ± 0.3 *‡ |
| Lung/body weight ratio | 5.2 ± 0.2 | n.d. | 11.1 1.1 * |
| Liver/body weight ratio | 49 ± 2 | n.d. | 47 ± 3 |
| Left ventricular internal diameter in end diastole (mm) | 3.76 ± 0.21 | 3.64 ± 0.08 | 4.91 ± 019*‡ |
| Left ventricular internal diameter in end systole (mm) | 2.23 ± 0.24 | 2.22 ± 0.09 | 4.01 ± 0.23 *‡ |
| Left ventricular fractional shortening (%) | 40.0 ± 3.5 | 39.1 ± 1.5 | 18.5 ± 2.1 *‡ |
| Diastolic ventricular septum wall thickness (mm) | 0.73 ± 0.01 | 0.92 ± 0.04 | 0.85 ± 0.08 ‡ |
| Diastolic left ventricular posterior wall thickness (mm) | 0.67 ± 0.07 | 0.84 ± 0.05 | 0.79 ± 0.02 |
| Heartbeat (beats/min) | 541 ± 26 | 541 ± 16 | 548 ± 30 |

| | | | |
|---|---|---|---|
| In the above Table 3, n represents the number of samples, and the data is represented by an average ± standard deviation; n.d. shows that measurement was not performed; * P < 0.01 versus the sham-operated same genotype mice; † P < 0.05 versus the control WT mouse fourweeks after TAC; and ‡ P < 0.05 versus the same genotype mice one week after TAC. | | | |

### (Histological investigation)

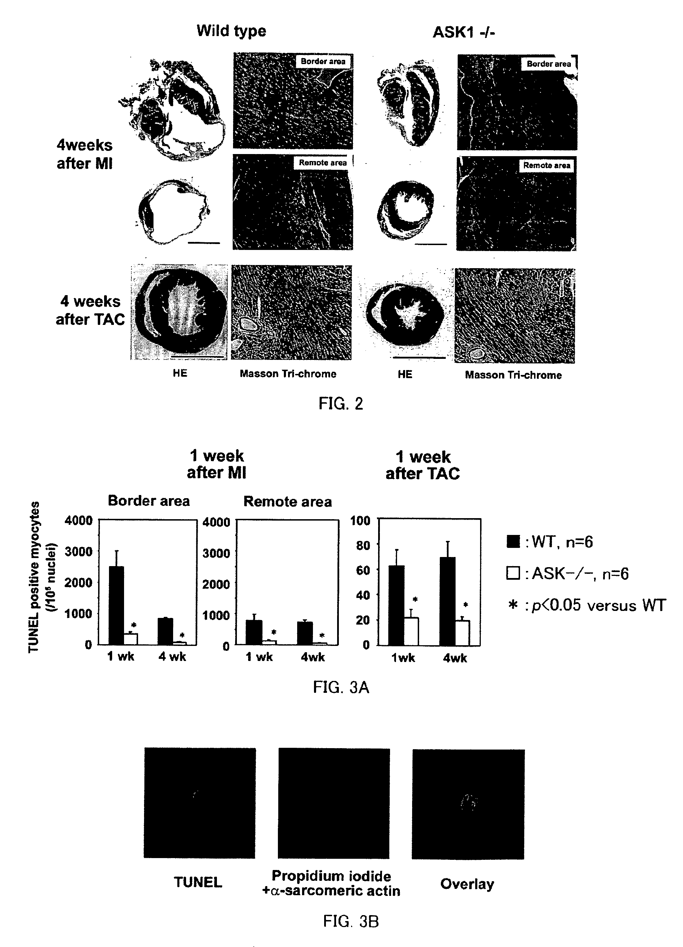

The hearts of ASK1 knockout mice and control WT mice four weeks after the above-mentioned coronary artery ligation were subjected to histological investigation. One example of the results is shown in an upper section of FIG. 2. In FIG. 2, a right column shows the heart of the ASK1 knockout mouse, and a left column shows the heart of the control WT mouse. Furthermore, in each column, a left side shows a section subjected to hematoxylin and eosin (HE) staining, a right side shows a section subjected to Masson's trichrome staining, and a bar shows 5 mm. As shown in FIG. 2, the heart of the control WT mouse four weeks after the treatment was clearly larger compared with that of the ASK1 knockout mouse. Furthermore, it was read from FIG. 2 that a part of an area remote from ischemic injury was replaced by a fibrous tissue in the control WT mouse, and the remote area was intact in the ASK1 knockout mouse. In a surviving portion of the left ventricle of the heart of the control WT mouse, intermuscular and perivascular fibrosis was recognized. However, perivascular fibrosis was observed only slightly in the remote area of the ASK1 knockout mouse.

Next, the hearts of the ASK1 knockout mice and the control WT mice four weeks after the TAC treatment were subjected to histological investigation. One example of the results is shown in a lower section of FIG. 2. In FIG. 2, a right column shows the heart of the ASK1 knockout mouse, and a left column shows the heart of the control WT mouse. Furthermore, in each column, a left side shows a section subjected to hematoxylin and eosin (HE) staining, and a right side shows a section subjected to Masson's trichrome staining. The dilation of the heart of the control WT mouse four weeks after the treatment was clear; however, the dilation of the ventricle and the atrium did not occur in the ASK1 knockout mouse. Furthermore, in both the mice, intermuscular fibrosis was observed in a scattered manner in the same way as in perivascular fibrosis, and the degree of intermuscular fibrous was the same as those of both the mice.

### (Mechanical stress)

The main stimulus of the myocardial remodeling was mechanical overloading. Therefore, the degree of mechanical stress loaded to the hearts of the control WT and the ASK1 knockout mice after the coronary artery ligation or TAC treatment was evaluated.

In a myocardial infarction model, the mechanical overload imposed on the surviving myocardium is estimated as a double product. One week after the coronary artery ligation, no significant difference was recognized in a LV systolic pressure and a heartbeat between the ASK1 knockout mice and the control WT mice (systolic pressure was 110.2 ± 6.0 mmHg in the ASK1 knockout mice, and was 108.2 ± 2.8 mmHg in the control WT mice. The heartbeat was 582 ± 10/min in the ASK1 knockout mice, and 559 ± 24/min in the control WT mice). Furthermore, the size of the infarct was similar in both the mice investigated immediately after the coronary artery ligation, the average of the ASK1 knockout mice was 51.3 ± 5.7 %, and the average of the control WT mice was 49.1 ± 4.9 %. Thus, it was suggested that the possibility of the phenotype of removal of ASK1 being related to the development of collateral circulation is low.

The mechanical stress produced by the TAC treatment can be estimated by measuring *in vivo* trans-stenotic pressure gradients seven days after the TAC. As a result, the TAC treatment remarkably increased the pressure gradient between two carotid arteries; however, no significant difference was recognized between the ASK1 knockout mice and the control WT mice (55.5 ± 5.7 mmHg in the ASK1 knockout mice, and 57.3 ± 4.6 mmHg in the control WT).

### (Apoptosis in left ventricular remodeling)

The effect of ASK1 removal with respect to apoptosis that increases in cardiomyocytes during left ventricular remodeling was evaluated. In order to count the number of cardiomyocytes in which apoptosis was induced after the coronary artery ligation or TAC treatment, TUNEL assay was used. One example of the results obtained by comparing the ASK1 knockout mice with the control WT mice is shown in FIG. 3. A graph in FIG. 3A shows one example of the relative number of TUNEL positive cells in a boundary area and a remote area (two left panels) one or four weeks after the coronary artery ligation, and in the myocardium (right panel) one or four weeks after the TAC treatment. As shown in FIG. 3A, one and four weeks after the coronary artery ligation, in the myocardium remote from the area of ischemic injury as well as in the boundary area of infarct, the number of TUNEL positive cells (i.e., the number of apoptosis cells) increased more remarkably in the control WT mice than in the ASK1 knockout mice. Furthermore, the TUNEL positive cells after the TAC treatment were unevenly distributed in the left ventricle wall, and the number thereof increased more remarkably in the control WT mice than in the ASK1 knockout mice.

It was confirmed by trichrome staining that the TUNEL positive cells are cardiomyocytes. One example of the results is shown in FIG. 3B. As shown in FIG. 3B, TUNEL staining looks green, and anti-alpha-sarcomere antibody and propidium iodide staining looks red. Thus, if the staining looks yellow when both the figures are overlapped with each other, cardiomyocytes can be confirmed.

### (Induction of apoptosis by activation of ASK1 protein)

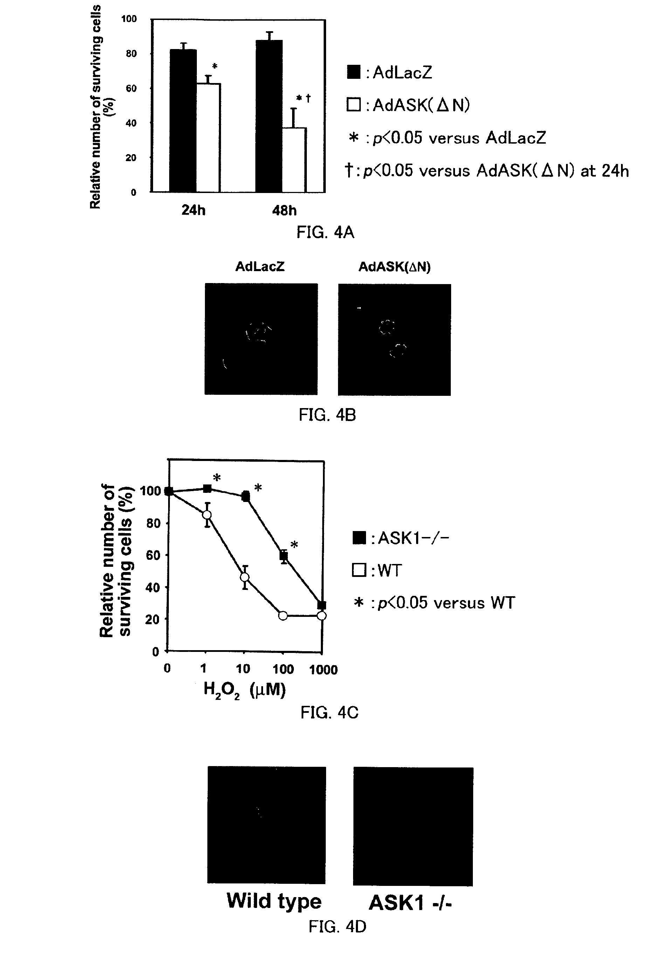

In order to confirm that ASK1 protein can induce apoptosis in cardiomyocytes, cardiomyocytes of neonatal rats were isolated, and adenovirus that expresses a constitutively active mutant ASK1 protein (AdASK(ΔN)) or β-galactosidase (AdLacZ) was infected at a multiplicity of infection of 100 plaque forming unit per cell. FIG. 4 shows the results. FIG. 4A is a graph of a cell survival rate represented in terms of percent with respect to the surviving number on the 0th day of infection with the virus. As shown in FIG. 4A, the overexpression of constitutively active mutant of ASK1 protein caused a decrease in the number of surviving cells.

On the other hand, even if β-galactosidase was expressed, no decrease in the number of surviving cells was recognized. FIG. 4B is a microphotograph showing cells 48 hours after the virus infection was subjected to Hoechst staining. As shown in FIG. 4B, when Hoechst33258 staining was performed after allowing a constitutively active mutant of ASK1 protein to be overexpressed, condensed nuclei chromatin of various degrees, and fragmented nuclei were observed. On the other hand, even when β-galactosidase was expressed, such nuclei were not observed. Furthermore, when the variable ASK1 protein was expressed with the multiplicity of infection of 10 plaque forming unit per cell, resulting in cardiomyocyte hypertrophy.

### (Necessity of ASK1 in apoptosis induced by H₂O₂)

Next, the necessity of ASK1 in apoptosis induced by H₂O₂ was confirmed using neonatal cardiomyocytes of ASK1 knockout mice (ASK^{-/-}) and control WT mice. The cardiomyocytes were cultured with H₂O₂ of various concentrations for 24 hours, and the number of surviving cells was measured by MTT assay, whereby the survival rate (%) of the cardiomyocytes was obtained. FIG. 4C shows the results. As shown in FIG. 4C, the resistance to H₂O₂ of the cardiomyocytes of ASK^{-/-} was more excellent than that of the cardiomyocytes of WT. Furthermore, FIG. 4D shows micrographs obtained by treating the cardiomyocytes with 10 µM of H₂O₂ for 24 hours, and subjecting the cardiomyocytes to Hoechst staining. As shown in FIG. 4D, it was confirmed from the form of the observed nuclei that apoptosis was induced by H₂O₂.

### (Activation of ASK1 protein after coronary artery ligation treatment and TAC treatment)

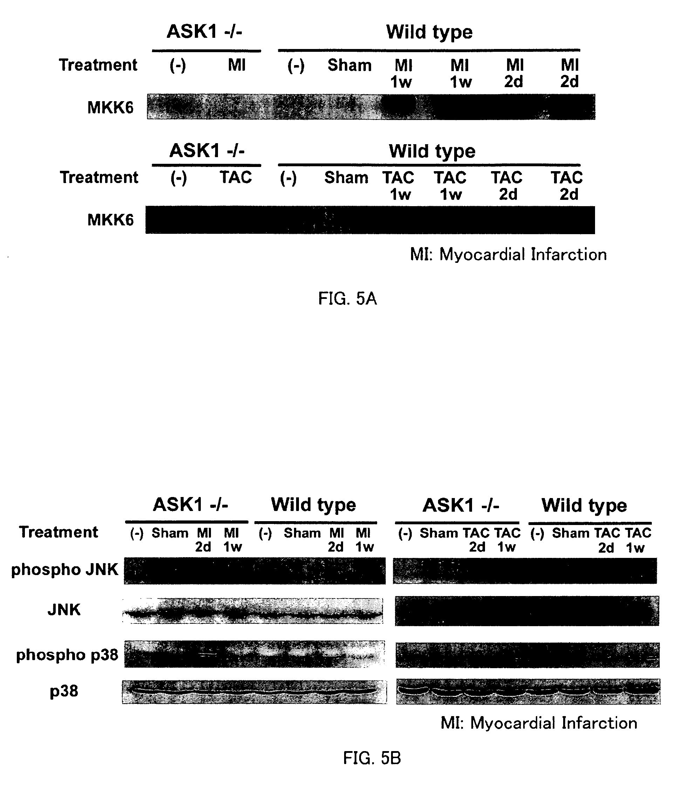

The activity of ASK1 protein during remodeling was confirmed. Regarding the heart after the coronary artery ligation or TAC treatment, the activity of ASK1 protein was measured. FIG. 5A shows the results. FIG. 5A shows measurement results of immune complex assay using His-MKK6 as a substrate. An upper panel shows the activation of ASK1 protein two days and one week after the coronary artery ligation treatment, and a lower panel shows the activation of ASK1 protein two days and one week after the TAC treatment. In the upper panel, myocardium homogenate was used, which was extracted from treated and untreated ASK1 knockout mice, untreated WT mice, WT mice subjected to a sham operation, and WT mice two days and one week after the treatment. In the lower panel, myocardium homogenate was used, which was extracted from ASK1 knockout mice two days and one week after the treatment, and WT mice subjected to a sham operation and WT mice two days and one week after the treatment. As shown in FIG. 5A, it was confirmed that, in the heart of the WT mice, ASK1 protein is activated remarkably after the coronary artery ligation and TAC treatment. However, in the ASK1 knockout mice, no significant activation of ASK1 was recognized.

Furthermore, during remodeling, in order to evaluate the activity of JNK and p38 present on a downstream of ASK1 protein, each phosphorylated state was measured using Western blot. FIG. 5B shows the results. FIG. 5B shows one example of the results of Western blot with respect to JNK and p38 of heart cells of the ASK1 knockout mice and the WT mice after the coronary artery ligation (left side) or the TAC treatment (right side). As shown in FIG. 5B, the activation of JNK and p38 was recognized after the coronary artery ligation and the TAC treatment in the hearts of the WT mice. On the other hand, in the hearts of the ASK1 knockout mice, although the activation of p38 was observed to the same degree as that of the WT mice, the activation of JNK was suppressed significantly. Thus, in remodeling, it was suggested that JNK plays an important role as a downstream factor of ASK1.

### Industrial Applicability

Chronic cardiac failure is one of the main causes of death in the developed countries. Furthermore, acute coronary artery disease has been overcome by the advancement of CCU (coronary artery disease intensive treatment) and coronary artery formation. This increases the occurrence of cardiac failure after myocardial infarction. Furthermore, a number of patients of cardiac failure also are found in a group of hypertension. The present invention is useful, for example, in the field of a drug for at least one of the prevention and the treatment of cardiac failure and in the field of a method for at least one of the prevention and the treatment of cardiac failure, which are expected to be demanded greatly.

## Claims

1. A drug for at least one of prevention and treatment of cardiac failure, comprising a compound that inhibits a functional expression of ASK1 protein in a cardiomyocyte as an active ingredient.

2. A drug for at least one of prevention and treatment of cardiac failure, comprising a compound that suppresses apoptosis of a cardiomyocyte induced by ASK1 protein as an active ingredient.

3. A drug for at least one of prevention and treatment of cardiac failure, comprising a compound that inhibits at least one selected from the group consisting of Daxx, TRAF2, calmodulin-dependent kinase II, MKK3, MKK4, MKK6, MKK7, JNK, and p38 MAPK as an active ingredient.

4. A drug for at least one of prevention and treatment of cardiac failure, comprising at least one kind of compound selected from the group consisting of a compound that inhibits kinase activity of ASK1 protein in a cardiomyocyte, a compound that inhibits translation of ASK1 mRNA in a cardiomyocyte, and a compound that inhibits transcription of ASK1 gene in a cardiomyocyte.

5. The drug according to claim 4, wherein the compound that inhibits kinase activity of ASK1 protein in a cardiomyocyte is at least one kind selected from the group consisting of a dominant negative mutant of ASK1 protein, an anti-ASK1 antibody, and thioredoxin.

6. The drug according to claim 4, wherein the compound that inhibits translation of ASK1 mRNA in a cardiomyocyte is at least one kind selected from antisense DNA, antisense RNA, and RNA for RNA interference.

7. A method for screening a drug for at least one of prevention and treatment of cardiac failure, comprising selecting a medicinal component for at least one of prevention and treatment of cardiac failure from a drug candidate compound by using inhibition of a functional expression of ASK1 protein as an indication.

8. A method for screening a drug for at least one of prevention and treatment of cardiac failure, comprising selecting a medicinal component for at least one of prevention and treatment of cardiac failure from a drug candidate compound by using suppression of apoptosis induced by ASK1 protein as an indication.

9. A method for screening a drug for at least one of prevention and treatment of cardiac failure, comprising selecting a medicinal component for at least one of prevention and treatment of cardiac failure from a drug candidate compound by using inhibition of kinase activity of ASK1 protein as an indication.

10. A method for screening a drug for at least one of prevention and treatment of cardiac failure, comprising selecting a medicinal component for at least one of prevention and treatment of cardiac failure from a drug candidate compound by using inhibition of autophosphorylation of ASK1 protein as an indication.

11. A method for screening a drug for at least one of prevention and treatment of cardiac failure, comprising selecting a medicinal component for at least one of prevention and treatment of cardiac failure from a drug candidate compound by using inhibition of transcription translation of ASK1 gene as an indication.

12. A method for screening a drug for at least one of prevention and treatment of cardiac failure, comprising selecting a medicinal component for at least one of prevention and treatment of cardiac failure from a drug candidate compound by using inhibition of activity of a factor activating ASK1 protein as an indication.

13. The method for screening according to claim 12, wherein the factor activating ASK1 protein is at least one selected from the group consisting of Daxx, TRAF2, and calmodulin-dependent kinase II.

14. A method for screening a drug for at least one of prevention and treatment of cardiac failure, comprising selecting a medicinal component for at least one of prevention and treatment of cardiac failure from a drug candidate compound by using inhibition of a factor activated by ASK1 protein as an indication.

15. The method for screening according to claim 14, wherein the factor activated by ASK1 protein is at least one selected from the group consisting of MKK3, MKK4, MKK6, MKK7, JNK, and p38 MAPK.

16. A method for at least one of prevention and treatment of cardiac failure, comprising inhibiting a functional expression of ASK1 protein in a cardiomyocyte.

17. A method for at least one of prevention and treatment of cardiac failure, comprising suppressing apoptosis of a cardiomyocyte induced by ASK1 protein.

18. A method for at least one of prevention and treatment of cardiac failure, comprising inhibiting at least one of kinase activity of ASK1 protein in a cardiomyocyte, autophosphorylation of the ASK1 protein in a cardiomyocyte, and transcription translation of ASK1 gene in a cardiomyocyte.

19. A method for at least one of prevention and treatment of cardiac failure, comprising administering a pharmaceutically acceptable effective amount of the drug for at least one of prevention and treatment of any one of claims 1 to 6.

20. A method for diagnosing cardiac failure, comprising measuring kinase activity or autophosphorylation of ASK1 protein in a cardiomyocyte.
